# EUROPEAN PATENT SPECIFICATION

(11) **EP 0 734 392 B1**
(45) Date of publication and mention of the grant of the patent: **16.12.1998**
(21) Application number: 95900414.4
(22) Date of filing: 27.10.1994
(51) Int. Cl.: C07J 9/00

(54) **CONVERSION OF BISNORALCOHOL TO BISNORALDEHYDE**
VERFAHREN ZUR HERSTELLUNG VON BISNORALDEHYDEN AUS BISNORALKOHOL
TRANSFORMATION DE BISNORALCOOL EN BISNORALDEHYDE

(30) Priority: 17.12.1993 US 168961
(43) Date of publication of application: 02.10.1996
(73) Proprietor: PHARMACIA & UPJOHN COMPANY, Kalamazoo, Michigan 49001 (US)
(72) Inventor: HEWITT, Bradley, Dee, Kalamazoo, MI 49004 (US)
(74) Representative: Perry, Robert Edward
(86) International application number: US9412196
(87) International publication number: WO9516698

(56) References cited:
- DE-A- 4 236 887
- JOURNAL OF ORGANIC CHEMISTRY., vol.52, no.12, 12 June 1987, EASTON US pages 2559 - 2562 P. N. ANELLI ET AL 'Fast and Selective Oxidation of Primary Alcohols to Aldehydes or to Carboxylic Acids and of Secondary Alcohols to Ketones Mediated by Oxoammonium salts under Two-Phase Conditions' cited in the application
- DATABASE WPI Section Ch, Week 8202, Derwent Publications Ltd., London, GB; Class B01, AN 82-03202E & JP,A,56 152 498 (MITSUBISHI CHEM IND KK) 26 November 1981 cited in the application
- BULLETIN OF THE CHEMICAL SOCIETY OF JAPAN., vol.58, no.3, 1985, TOKYO JP pages 1081 - 1082 I. NITTA ET AL 'The Synthesis of the Corticoid Side-Chain. II. A New Synthesis of 17-alpha,21-Dihydroxypregna-1,4-diene-3 ,20-dione 21-Acetate from 21-Hydroxy-20-me thylpregna-1,4-dien-3,20-dione'
- JOURNAL OF THE AMERICAN CHEMICAL SOCIETY., vol.106, no.11, 30 May 1984, GASTON, PA US pages 3374 - 3376 M. F. SEMMELHACK ET AL 'Oxidation of Alcohols to Aldehydes with Oxygen and Cupric Ion, Mediated by Nitrosonium Ion' cited in the application
- CHEMICAL ABSTRACTS, vol. 122, no. 1, 2 January 1995, Columbus, Ohio, US; abstract no. 9868, J. SUZUKI 'Preparation of Ether-Bond Containing Aldehydes' page 1099 ;column 2 ;

## Description

### 1. Field of the Invention

The present invention is a process for the conversion of bisnoralcohol (I) to bisnoraldehyde (II) which is a known intermediate in the synthesis of progesterone.

### 2. Description of the Related Art

The oxidation of bisnoralcohol (I) to bisnoraldehyde (II) is a well known process.

4-hydroxy-TEMPO (4-hydroxy-2,2,6,6-tetramethylpiperidine- 1-oxyl) is known, see *Synthesis,* 190-202 and 401-414 (1971).

*J. Org. Chem.,* 52, 2559 (1987) discloses TEMPO and 4-Methoxy-TEMPO catalyzed, two-phase oxidation of primary alcohols and secondary alcohols to aldehydes and ketones, respectively, using potassium bromide and 0.35 M sodium hypochlorite buffered to Ph 8.5 with sodium bicarbonate.

*J. Org. Chem.,* 56, 6110 (1991) discloses the use of stoichiometric amounts of oxammonium salts, generated by treatment of TEMPO or 4-acetylamino-TEMPO with organic sulfonic acids, for the selective oxidation of primary or secondary alcohols to aldehydes or ketones, respectively.

*J. Am. Chem. Soc.,* 106, 3374 (1984) discloses the use of TEMPO or 4-hydroxy-TEMPO to catalyze the oxidation of primary or secondary alcohols to aldehydes or ketones, respectively, by oxygen and copper (II) salts.

US Patent 5,136,102 discloses the use of TEMPO or 4-substituted TEMPO derivatives and a bromide containing salt to catalyze the oxidation of secondary alcohols to ketones with nitric acid and oxygen.

US Patent 5,155,278 discloses the use of TEMPO or 4-substituted TEMPO derivatives to catalyze the oxidation of primary alcohols to aldehydes with nitric acid and oxygen.

US Patent 5,155,279 discloses the use of TEMPO or 4-substituted TEMPO derivatives to catalyze the selective oxidation of primary alcohols to aldehydes with nitric acid in the absence of oxygen.

US Patent 5,155,280 discloses the use of TEMPO or 4-substituted TEMPO derivatives and an alkali metal nitrosodisulfonate salt to catalyze the selective oxidation of primary alcohols to aldehydes with oxygen in the absence of nitric acid.

Japanese patent J5 6152498 discloses the oxidation of bisnoralcohol to bisnoraldehyde using dimethyl sulfide and N-chlorosuccinimde or chlorine.

### SUMMARY OF INVENTION

Disclosed is a process for the production bisnoraldehyde (II) 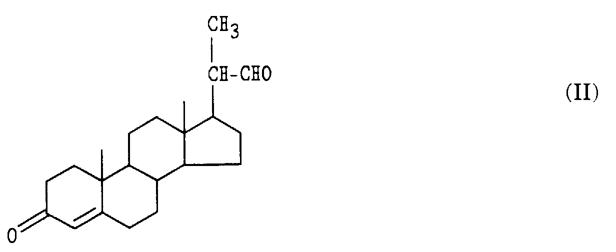 which comprises:
(1) forming a mixture of
   (a) bisnoralcohol (I) 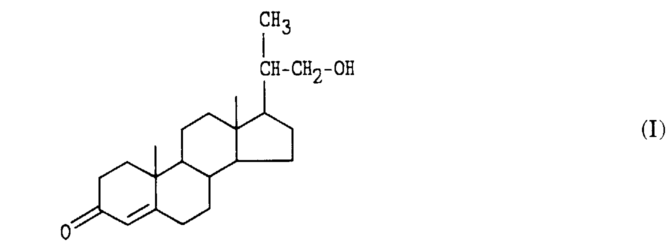
   (b) a catalytic amount of 4-hydroxy-2,2,6,6-tetramethylpiperidine-1-oxyl in a pH range of about 8.5 to about 10.5 in a temperature range of about - 10° to about 15°, and
(2) contacting the mixture of step (1) with a stoichiometric amount of hypochlorite.

### DETAILED DESCRIPTION OF THE INVENTION

Bisnoraldehyde (II) is known to be useful as an intermediate in the synthesis of progesterone and hydrocortisone, see *J. Am. Chem. Soc.,* 74, 5933 (1952).

The present invention is practiced by (1) forming a mixture of bisnoralcohol (I), a catalytic amount of 4-hydroxy TEMPO in a pH range of about 8.5 to about 10.5 and in a temperature range of about - 10° to about 15°, and (2) contacting the mixture of step (1) with a stoichiometric amount of hypochlorite. It is preferred to perform the reaction in the presence of bromide, preferably a catalytic amount of the bromide. The mixture can be cooled at any point prior to the addition of the hypochlorite.

Operable amounts of the 4-hydroxy-TEMPO are from about 0.025 mole percent to about 15 mole percent; it is preferred that the amount of the 4-hydroxy-TEMPO be from about 0.025 mole percent to about 2.5 mole percent. Operable amounts of the bromide are from about 5 mole percent to about 25 mole percent; it is preferred that the amount of bromide be from about 10 mole percent to about 15 mole percent. The pH is preferably regulated by the use of bicarbonate. Operable amounts of bicarbonate are from about 5 mole percent to about 30 mole percent; it is preferred that the amount of bicarbonate be from about 10 mole percent to about 20 mole percent. The cation of the bromide or bicarbonate is not important as long as it is soluble; preferred cation are sodium, potassium and lithium, more preferably sodium or potassium. Operable solvents include dichloroethane, toluene, ethyl acetate, methyl tert-butyl ether, dichloromethane, o-dichlorobenzene chlorobenzene and chloroform; it is preferred that the solvent be methylene chloride. While the operable solvents are organic water immiscible solvents, a small amount of water is operable and even preferred as is known to those skilled in the art. In addition, the hypochlorite is added as an aqueous mixture. It is preferred that the reaction temperature be in the range of about - 5° to about 5°. It is preferred that the hypochlorite is added over a period of from about 1 hr to about 6 hr. It is preferred that the amount of hypochlorite be from about 95 mole percent to about 120 mole percent. Following step (2) it is preferred to quench the reaction mixture. Operable quenching agents include bisulfite, thiosulfate, dimethylsulfide, trimethylphosphate and triethylphosphate; it is preferred that the quenching agent be sodium or potassium thiosulfate.

The process of the present invention can be practiced in either batch mode or continuous mode as is known to those skilled in the art.

The reaction mixture is worked up by methods well known to those skilled in the art.

The bisnoraldehyde (II) can be transformed to progesterone by known methods, see J. *C.* S. *Chem. Comm.,* 314 (1969) and *Tet. Lett.,* 985 (1969).

### DEFINITIONS AND CONVENTIONS

The definitions and explanations below are for the terms as used throughout this entire document including both the specification and the claims.

### DEFINITIONS

All temperatures are in degrees Centigrade.

4-hydroxy-TEMPO refers to 4-hydroxy-2,2,6,6-tetramethylpiperidine-1-oxyl.

TEMPO refers to 2,2,6,6-tetramethylpiperidine- 1-oxyl.

### EXAMPLES

Without further elaboration, it is believed that one skilled in the art can, using the preceding description, practice the present invention to its fullest extent. The following detailed examples describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

### EXAMPLE 1 Bisnoralcohol (I) to Bisnoraldehyde (II) at 1° with 4-Hydroxy-TEMPO

A mixture of bisnoralcohol (I, 4 g), 4-hydroxy-2,2,6,6-tetramethylpiperidine-1-oxyl (4-hydroxy-TEMPO, 10 mg), potassium bromide (133 mg), sodium bicarbonate (133 mg), dichloromethane (14 ml) and water (2.2 ml) are cooled to 1°. While maintaining this temperature, aqueous sodium hypochlorite (14%, 6.3 ml) is added over a five hr period. The reaction is complete and aqueous sodium thiosulfate is added, the two phases were separated, and the bisnoraldehyde product is crystallized by replacing the dichloromethane with heptane to give the title compound, mp = 153-154°; NMR (CDCl₃) 9.56, 5.73, 2.2-2.5, 1.2-2.1, 1.20, 1.10, 0.79 δ; [α]_{D}²² = + 83.4° (methylene chloride, c = 1).

### EXAMPLE 2 Bisnoralcohol (I) to Bisnoraldehyde (II) at 10° with 4-Hydroxy-TEMPO

Following the general procedure of EXAMPLE 1 and making non-critical variations the process of EXAMPLE 1 is repeated at 10° and the title compound is obtained.

### EXAMPLE 3 Bisnoralcohol (I) to Bisnoraldehyde (II) at -10° with 4-Hydroxy-TEMPO

Following the general procedure of EXAMPLE 1 and making non-critical variations the process of EXAMPLE 1 is repeated at -10° and the title compound is obtained.

### EXAMPLE 4 Bisnoralcohol (I) to Bisnoraldehyde (II) at 1° with 4-Hydroxy-TEMPO

Following the general procedure of EXAMPLE 1 and making non-critical variations the process of EXAMPLE 1 is repeated using 500 mg of 4-hydroxy-TEMPO and the title compound is obtained.

### EXAMPLE 5 Bisnoralcohol (I) to Bisnoraldehyde (II) at 1° with 4-Hydroxy-TEMPO

Following the general procedure of EXAMPLE 1 and making non-critical variations the process of EXAMPLE 1 is repeated using 5 mg of 4-hydroxy-TEMPO and the title compound is obtained.

### EXAMPLE 6 Bisnoralcohol (I) to Bisnoraldehyde (II) with 4-oxo-TEMPO

A mixture of bisnoralcohol (I, 6.6 g), 4-oxo-2,2,6,6-tetramethylpiperidine-1-oxyl (18 mg), dichloromethane (30 ml), sodium bicarbonate (180 mg), potassium bromide (238 mg) and water (5 ml) is cooled to 1°. Then aqueous sodium hypochlorite (14.6 %, 11.4 ml) is added to the mixture over a 15 min period. The reaction produced the title compound but in only a 7% conversion of bisnoralcohol with 58% selectivity for bisnoraldehyde.

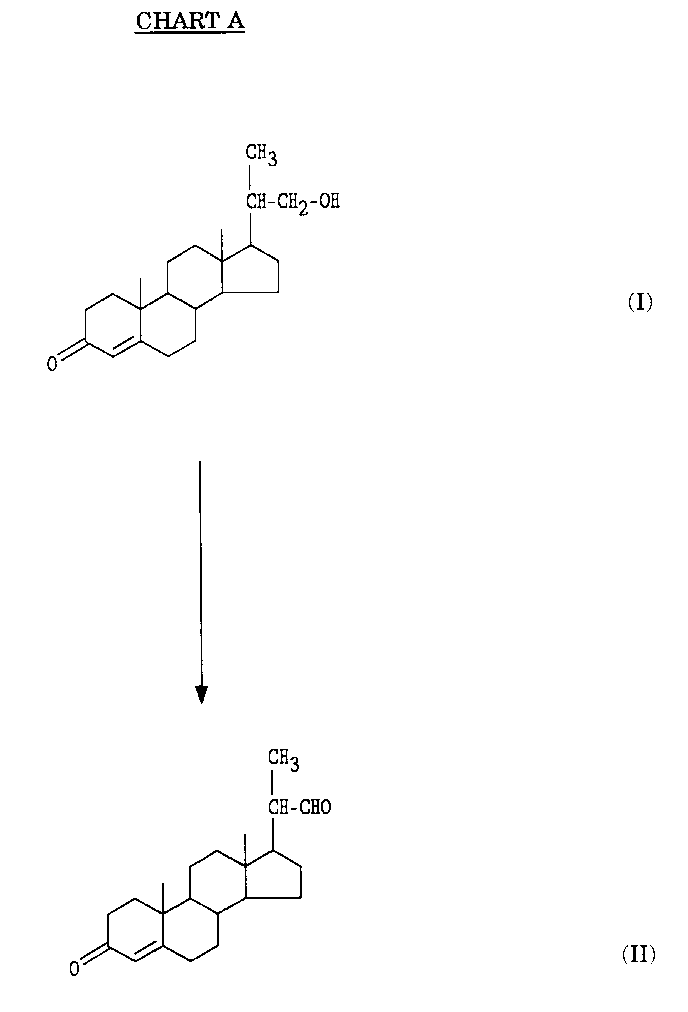

## Claims

1. A process for the production of bisnoraldehyde, i.e. pregn-4-en-3-one-20-carboxaldehyde, which comprises contacting a mixture of bisnoralcohol, i.e. 20-hydroxymethylpregn-4-en-3-one, and a catalytic amount of 4-hydroxy-2,2,6,6-tetramethylpiperidine 1-oxide, at a pH of 8.5 to 10.5 and at a temperature of -10° to 15°C, with a stoichiometric amount of hypochlorite.

2. A process according to claim 1, where the catalytic amount of the 4-hydroxy-2,2,6,6-tetramethylpiperidine 1-oxide is from 0.025 to 15 mole %.

3. A process according to claim 2, where the catalytic amount of the 4-hydroxy-2,2,6,6-tetramethylpiperidine 1-oxide is from 0.025 to 2.5 mole %.

4. A process according to any preceding claim, which is performed in the presence of a catalytic amount of bromide.

5. A process according to claim 4, where the catalytic amount of bromide is from 5 to 25 mole %.

6. A process according to claim 5, where the catalytic amount of bromide is from 10 to 15 mole %.

7. A process according to any preceding claim, where the pH is regulated by the presence of bicarbonate.

8. A process according to claim 7, where the amount of bicarbonate is from 5 to 30 mole %.

9. A process according to claim 8, where the amount of bicarbonate is from 10 to 20 mole %.

10. A process according to any of claims 4 to 6 and any of claims 7 to 9, where the bisnoralcohol, the 4-hydroxy-2,2,6,6-tetramethylpiperidine 1-oxide, the bromide and the bicarbonate are all mixed together, and then cooled to -10° to 15°C.

11. A process according to any preceding claim, which is performed in the presence of a solvent selected from dichloroethane, toluene, ethyl acetate, methyl tert-butyl ether, dichloromethane, o-dichlorobenzene, chlorobenzene and chloroform.

12. A process according to claim 11, where the solvent is methylene chloride.

13. A process according to any preceding claim, where the temperature is -5 to 5°C.

14. A process according to any preceding claim, where the amount of hypochlorite is from 95 to 120 mole %.

15. A process according to any preceding claim, where the reaction mixture is quenched, after contacting said mixture with the hypochlorite.

16. A process according to claim 15, where said reaction mixture is quenched with a quenching agent selected from bisulfite, thiosulfate, dimethylsulfide, trimethylphosphate and triethylphosphate.

17. A process according to claim 16, where the quenching agent is sodium or potassium thiosulfate.

## Patentansprüche

1. Verfahren zur Herstellung von Bisnoraldehyd, d.h. Pregn-4-en-3-on-20-carboxaldehyd, durch Inberührungbringen eines Gemisches aus Bisnoralkohol, d.h. 20-Hydroxymethylpregn-4-en-3-on, und einer katalytischen Menge von 4-Hydroxy-2,2,6,6-tetramethylpiperidin-1-oxid bei einem pH-Wert von 8,5 bis 10,5 und einer Temperatur von -10 bis 15°C mit einer stöchiometrischen Menge Hypochlorit.

2. Verfahren nach Anspruch 1, wobei die katalytische Menge an 4-Hydroxy-2,2,6,6-tetramethylpiperidin-1-oxid 0,025 bis 15 Mol-% beträgt.

3. Verfahren nach Anspruch 2, wobei die katalytische Menge an 4-Hydroxy-2,2,6,6-tetramethylpiperidin-1-oxid 0,025 bis 2,5 Mol-% beträgt.

4. Verfahren nach einem der vorhergehenden Ansprüche, das in Gegenwart einer katalytischen Menge Bromid durchgeführt wird.

5. Verfahren nach Anspruch 4, wobei die katalytische Menge Bromid 5 bis 25 Mol-% beträgt.

6. Verfahren nach Anspruch 5, wobei die katalytische Menge Bromid 10 bis 15 Mol-% beträgt.

7. Verfahren nach einem der vorhergehenden Ansprüche, wobei der pH-Wert durch die Anwesenheit von Bicarbonat gesteuert wird.

8. Verfahren nach Anspruch 7, wobei die Bicarbonatmenge 5 bis 30 Mol-% beträgt.

9. Verfahren nach Anspruch 8, wobei die Bicarbonatmenge 10 bis 20 Mol-% beträgt.

10. Verfahren nach einem der Ansprüche 4 bis 6 und einem der Ansprüche 7 bis 9, wobei der Bisnoralkohol, das 4-Hydroxy-2,2,6,6-tetramethylpiperidin-1-oxid, das Bromid und das Bicarbonat alle miteinander vermischt werden und anschließend die Mischung auf -10 bis 15°C abgekühlt wird.

11. Verfahren nach einem der vorhergehenden Ansprüche, das in Gegenwart eines Lösungsmittels durchgeführt wird, das unter Dichlorethan, Toluol, Ethylacetat, Methyltert.-butylether, Dichlormethan, o-Dichlorbenzol, Chlorbenzol und Chloroform ausgewählt ist.

12. Verfahren nach Anspruch 11, wobei das Lösungsmittel Methylenchlorid ist.

13. Verfahren nach einem der vorhergehenden Ansprüche, wobei die Temperatur -5 bis 5°C beträgt.

14. Verfahren nach einem der vorhergehenden Ansprüche, wobei die Hypochloritmenge 95 bis 120 Mol-% beträgt.

15. Verfahren nach einem der vorhergehenden Ansprüche, wobei das Reaktionsgemisch nach Inberührungbringen des Gemisches mit dem Hypochlorit gequencht wird.

16. Verfahren nach Anspruch 15, wobei das Reaktionsgemisch mit einem Quenchmittel, das aus Bisulfit, Thiosulfat, Dimethylsulfid, Trimethylphosphat und Triethylphosphat ausgewählt ist, gequencht wird.

17. Verfahren nach Anspruch 16, wobei das Quenchmittel Natrium- oder Kaliumthiosulfat ist.

## Revendications

1. Procédé pour la production de bisnoraldéhyde, c'est-à-dire de prégn-4-ène-3-one-20-carboxaldéhyde, qui comprend la mise en contact d'un mélange de bisnoralcool, c'est-à-dire de 20-hydroxyméthylprégn-4-ène-3-one, et d'une quantité catalytique de 1-oxyde de 4-hydroxy-2,2,6,6-tétraméthylpipéridine, à un pH de 8,5 à 10,5 et à une température de -10° à 15°C, avec une quantité stoechiométrique d'un hypochlorite.

2. Procédé suivant la revendication 1, dans lequel la quantité catalytique du 1-oxyde de 4-hydroxy-2,2,6,6-tétraméthylpipéridine est comprise dans l'intervalle de 0,025 à 15 moles %.

3. Procédé suivant la revendication 2, dans lequel la quantité catalytique du 1-oxyde de 4-hydroxy-2,2,6,6-tétraméthylpipéridine est comprise dans l'intervalle de 0,025 à 2,5 moles %.

4. Procédé suivant l'une quelconque des revendications précédentes, qui est mis en oeuvre en présence d'une quantité catalytique d'un bromure.

5. Procédé suivant la revendication 4, dans lequel la quantité catalytique de bromure est comprise dans l'intervalle de 5 à 25 moles %.

6. Procédé suivant la revendication 5, dans lequel la quantité catalytique de bromure est comprise dans l'intervalle de 10 à 15 moles %.

7. Procédé suivant l'une quelconque des revendications précédentes, dans lequel le pH est régulé par la présence d'un bicarbonate.

8. Procédé suivant la revendication 7, dans lequel la quantité de bicarbonate est comprise dans l'intervalle de 5 à 30 moles %.

9. Procédé suivant la revendication 8, dans lequel la quantité de bicarbonate est comprise dans l'intervalle de 10 à 20 moles %.

10. Procédé suivant l'une quelconque des revendications 4 à 6 et l'une quelconque des revendications 7 à 9, dans lequel le bisnoralcool, le 1-oxyde de 4-hydroxy-2,2,6,6-tétraméthylpipéridine, le bromure et le bicarbonate sont tous mélangés les uns aux autres, puis le mélange est refroidi à une température de -10° à 15°C.

11. Procédé suivant l'une quelconque des revendications précédentes, qui est mis en oeuvre en présence d'un solvant choisi entre le dichloréthane, le toluène, l'acétate d'éthyle, l'éther de méthyle et tertio-butyle, le dichlorométhane, le o-dichlorobenzène, le chlorobenzène et le chloroforme.

12. Procédé suivant la revendication 11, dans lequel le solvant consiste en chlorure de méthylène.

13. Procédé suivant l'une quelconque des revendications précédentes, dans lequel la température est comprise dans l'intervalle de -5 à 5°C.

14. Procédé suivant l'une quelconque des revendications précédentes, dans lequel la quantité d'hypochlorite est comprise dans l'intervalle de 95 à 120 moles %.

15. Procédé suivant l'une quelconque des revendications précédentes, dans lequel le mélange réactionnel est désactivé, après la mise en contact dudit mélange avec l'hypochlorite.

16. Procédé suivant la revendication 15, dans lequel le mélange réactionnel est désactivé avec un agent de désactivation choisi entre un bisulfite, un thiosulfate, le sulfure de diméthyle, le phosphate de triméthyle et le phosphate de triéthyle.

17. Procédé suivant la revendication 16, dans lequel l'agent de désactivation consiste en thiosulfate de sodium ou de potassium.
